(19) Europäisches Patentamt
European Patent Office
Office européen des brevets

(11) Veröffentlichungsnummer: **0 074 071**
**A1**

(12) # EUROPÄISCHE PATENTANMELDUNG

(21) Anmeldenummer: 82108021.5

(22) Anmeldetag: 01.09.82

(51) Int. Cl.³: C 07 C 103/38
C 07 C 103/48, C 07 C 153/09
C 07 D 307/32, C 07 D 317/28
A 01 N 37/30, A 01 N 43/08

(30) Priorität: 09.09.81 DE 3135670

(43) Veröffentlichungstag der Anmeldung: 16.03.83 Patentblatt 83/11

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Rentzea, Costin, Dr.
Neuenheimer Landstrasse 72
D-6900 Heidelberg(DE)

(72) Erfinder: Thym, Sabine, Dr.
Hasenhain 20
D-6901 Dossenheim(DE)

(72) Erfinder: Graf, Hermann, Dr.
Ginsterstrasse 15
D-6704 Mutterstadt(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

(54) **Oxalamide, Verfahren zu ihrer Herstellung und diese enthaltende Fungizide.**

(57) Oxalamide der allgemeinen Formel I

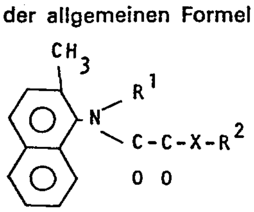

I,

worin

$R^1$ 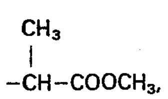, 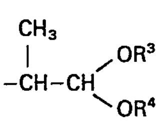 oder 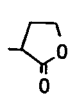

$R^2$ einen gegebenenfalls substituierten Alkyl-, Alkenyl- oder Cycloalkylrest bedeutet,

X für O oder S steht und

$R^3$ und $R^4$ Methyl oder Ethyl bedeuten oder $R^3$ und $R^4$ zusammen eine Alkylengruppe darstellen, die zusammen mit dem Rest

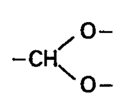

einen gegebenenfalls substituierten Dioxolan- oder 1,3-Dioxanring bilden und diese enthaltende Fungizide.

EP 0 074 071 A1

Oxalamide, Verfahren zu ihrer Herstellung und diese enthaltende Fungizide

Die vorliegende Erfindung betrifft neue N-substituierte 2-Methylnaphthylamide, Verfahren zu ihrer Herstellung und Fungizide, die diese Verbindungen enthalten.

Es ist bereits bekannt, Zink-ethylen-1,2-bisdithiocarbamat, das N-Trichlormethylthiophthalimid und das N-Trichlormethylthiotetrahydro-phthalimid als Fungizide in der Landwirtschaft und im Gartenbau zu verwenden. Die genannten Verbindungen sind gute Mittel zur Bekämpfung von pilzlichen Krankheiten (vgl. R. Wegler, "Chemie der Pflanzenschutz- und Schädlingsbekämpfungsmittel", Band 2, Seiten 65 bis 66 und 109 und Band 4, Seiten 139 und 191, Berlin/Heidelberg/New York, (1970) und (1977)).

Jedoch sind diese Fungizide nach erfolgter Infektion nicht verwendbar und ihre Wirkung bei niedrigen Aufwandkonzentrationen genügt nicht den Anforderungen der Praxis.

Es wurde nun gefunden, daß neue N-substituierte Oxalamide der allgemeinen Formel I

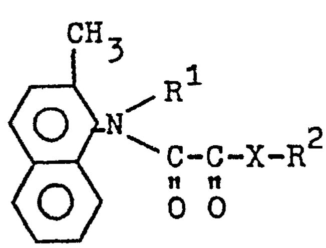

I,

worin

$R^1$ 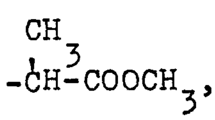, 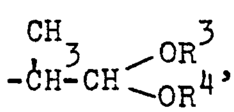, oder 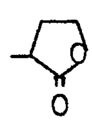 und

Sws/P

$R^2$ einen gegebenenfalls durch Methoxy, Ethoxy oder Halogen substituierten $C_1$-$C_6$-Alkyl-, $C_2$-$C_6$-Alkenyl- oder $C_5$-$C_6$-Cycloalkylrest bedeutet,

X für O oder S steht und

$R^3$ und $R^4$ Methyl oder Ethyl bedeuten oder $R^3$ und $R^4$ zusammen eine $C_2$-$C_3$-Alkylengruppe bedeuten, die zusammen mit dem Rest 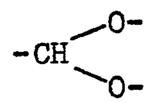 einen gegebenenfalls durch $C_1$-$C_3$-Alkyl substituierten Dioxolan- oder 1,3-Dioxanring bilden, starke fungizide Eigenschaften aufweisen.

In der Formel I steht $R^2$ bevorzugt für Methyl, Ethyl, n-Propyl, Isopropyl, n-Butyl, Isobutyl, sek.-Butyl, tert.-Butyl, n-Pentyl, Isoamyl, 2-Methoxyethyl, 2-Ethoxyethyl, 3-Methoxypropyl, 2-Chlorethyl, 2-Bromethyl, 2,2,2-Trifluorethyl, Allyl, 2-Buten-1-yl, Cyclopentyl oder Cyclohexyl.

Die neuen N-substituierten Oxalamide der Formel I besitzen ein chirales Kohlenstoffatom in dem Rest $R^1$ und je nach Beschaffenheit von $R^1$ noch weitere Chiralitätszentren. Nach üblichen Methoden können die optisch reinen Enantiomeren bzw. die Diastereomeren erhalten werden. Die vorliegende Erfindung erfaßt auch diese Verbindungen in reiner Form oder ihre Mischungen. Als Fungizide sind sowohl die reinen Enantiomeren bzw. die einheitlichen Diastereomeren als auch die bei der Synthese üblicherweise anfallenden Mischungen wirksam.

Weiterhin wurde gefunden, daß man die neuen Verbindungen der allgemeinen Formel (I) erhält, wenn man ein 2-Methylnaphthylamin der Formel II

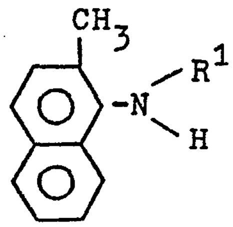

II,

in welcher

$R^1$ die obengenannte Bedeutung hat, mit einem Oxalsäurehalbesterchlorid der Formel III

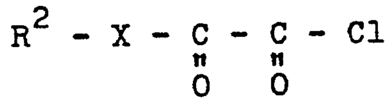

III,

in welcher

$R^2$ und X die oben angegebenen Bedeutungen haben,

gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen -10 und 100°C umsetzt. Als bevorzugte, gegenüber den Reaktionsteilnehmern inerte Lösungs- oder Verdünnungsmittel werden z.B. aliphatische oder aromatische Kohlenwasserstoffe wie Pentan, Cyclohexan, Petrolether, Benzol, Toluol oder Xylol; Halogenkohlenwasserstoffe, beispielsweise Methylenchlorid, Chloroform, 1,2-Dichlorethan, Chlorbenzol; Ketone wie Aceton und Methylethylketon; Ether wie Diethylether, Dimethoxyethan, Tetrahydrofuran oder Dioxan; Ester wie Essigsäureethylester; Nitrile wie Acetonitril; Sulfoxide wie Dimethylsulfoxid oder entsprechende Gemische verwendet.

Geeignete anorganische oder organische Basen, die gegebenenfalls auch als säurebindende Mittel bei der Reaktion verwendet werden können, sind beispielsweise Alkali- und

Erdalkalicarbonate, wie Natrium- oder Kaliumbicarbonat, Natrium- oder Kaliumcarbonat, Calciumcarbonat; Borate wie Natriumborat; Phosphate wie Natrium- oder Kaliumdi- oder -triphosphat oder Amine wie Triethylamin, N,N-Dimethylanilin, N,N-Dimethylcyclohexylamin, N-Methylpiperidin oder Pyridin. Es können aber auch andere übliche Basen verwendet werden.

Das erfindungsgemäße Herstellungsverfahren kann auch ohne säurebindende Mittel durchgeführt werden, wobei in einigen Fällen das Durchleiten von trockenem Stickstoff zur Vertreibung des entstandenen Chlorwasserstoffs angezeigt ist.

Als Reaktionsbeschleuniger kommen vorzugsweise Metallhalogenide, wie Natriumbromid oder Kaliumiodid, Azole wie Imidazol oder 1,2,4-Triazol, Pyridine wie 4-Dimethylaminopyridin oder Dimethylformamid in Frage.

Die erfindungsgemäßen Umsetzungen erfolgen beispielsweise bei Temperaturen zwischen -10 und +100°C, vorzugsweise zwischen 0 und +40°C, drucklos oder unter Druck, kontinuierlich oder diskontinuierlich.

Die Ausgangsstoffe der allgemeinen Formel II sind teilweise bekannt und werden nach allgemein bekannten Methoden synthetisiert.

Die Herstellung der neuen Oxalamide wird durch folgendes Beispiel erläutert:

Beispiel 1

Herstellung von

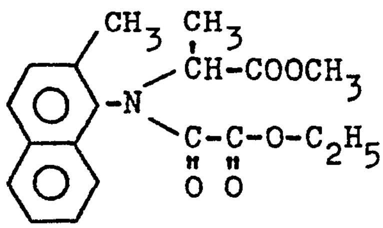

N-Ethoxalyl-N-(2-methyl-1-naphthyl)-alanin-methylester (Verbindung Nr. 1)

Einer Lösung von 19,4 g (0,08 Mol) N-(2-Methyl-1-naphthyl)-alanin-methylester in 100 ml trockenem Toluol werden 16,3 g (0,12 Mol) Ethoxalylchlorid in 20 ml Toluol bei 25°C zugetropft. Das Gemisch wird bei 80°C 8 Stunden nachgerührt. Dabei wird der entstehende Chlorwasserstoff mit einem schwachen Stickstoffstrom ständig aus dem Reaktionsgemisch vertrieben. Nach Abkühlen wird das Gemisch 1 Stunde mit der Lösung von 5 g Natriumhydrogencarbonat und 1 g Imidazol in 100 ml Wasser gerührt, die organische Phase getrennt, über $Na_2SO_4$ getrocknet, mit Kohle entfärbt und im Vakuum eingedampft. Der verbleibende ölige Rückstand wird 3 Stunden bei 50°C und 0,1 mbar getrocknet.

Man erhält 19,6 g (71,4 % d.Th.) der Verbindung Nr. 1 als hellgelbes Harz. IR-Spektrum (KBr): 3050, 2980, 1742, 1660, 1445, 1400, 1200, 1100, 1005, 985, 860, 815, 780, 745 $cm^{-1}$.

Entsprechend werden folgende Verbindungen hergestellt:

| Bsp. Nr. | $R^1$ | $R^2$ | X | physikal. Konstante oder IR-Spektrum [$cm^{-1}$] |
|---|---|---|---|---|
| 2 | $-CH(CH_3)-COOCH_3$ | $-CH_3$ | O | 3040,2940,1740,1662,1430, 1386,1220,1000,876,840, 812,742 |
| 3 | " | $-C_3H_7$-n | O | Harz |
| 4 | " | $-C_3H_7$-i | O | 3060,2990,1735,1660,1442, 1395,1200,1095,1040,930, 814,746 |
| 5 | " | $-C_4H_9$-n | O | Schmp. 68°C |
| 6 | " | $-C_4H_9$-i | O | Harz |
| 7 | " | $-C_4H_9$-sek. | O | Öl |
| 8 | " | $-C_4H_9$-tert. | O | Öl |
| 10 | " | $-C_5H_{11}$-n | O | 3045,2950,1740,1665,1400, 1370,1195,1108,1045,1010, 817,783,747 |
| 11 | " | $-C_5H_{11}$-i | O | Harz |
| 12 | " | $-CH_2-CH_2-Cl$ | O | 3040,2945,1745,1665,1448, 1404,1300,1200,1004,974, 817,745 |
| 13 | " | $-CH_2-CH_2-Br$ | O | Harz |
| 14 | " | $-C_3H_7$-n | S | Harz |
| 15 | " | $-C_3H_7$-i | S | Öl |
| 16 | " | $-C_4H_9$-n | S | Schmp. 62°C |
| 17 | " | $-C_4H_9$-i | S | Harz |
| 18 | " | $-C_5H_{11}$-n | S | 3040,2945,2920,1742,1660, 1450,1369,1200,1055,990, 978,814 |
| 19 | " | $-CH_2-CH=CH_2$ | O | Öl |
| 20 | " | $-CH_2-CH=CH-CH_3$ | O | Öl |
| 21 | " | Cyclopentyl | O | Harz |
| 22 | " | Cyclohexyl | O | Harz |

| Bsp. Nr. | $R^1$ | $R^2$ | $R^3$ | $R^4$ | X | physikal. Konstante oder IR-Spektrum [$cm^{-1}$] |
|---|---|---|---|---|---|---|
| 23 | 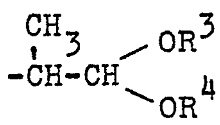 | $-CH_3$ | $-CH_3$ | $-CH_3$ | O | Öl |
| 24 | " | $-CH_3$ | $-CH_2-CH_2-$ | | O | |
| 25 | " | $-C_2H_5$ | $-CH_3$ | $-CH_3$ | O | Öl |
| 26 | " | $-C_2H_5$ | $-CH_2-CH_2-$ | | O | Harz |
| 27 | " | $i-C_3H_7$ | $-CH_3$ | $-CH_3$ | S | |
| 28 | " | $n-C_4H_9$ | $-CH_3$ | $-CH_3$ | O | Öl |
| 29 | " | $n-C_4H_9$ | $-CH_3$ | $-CH_3$ | S | Öl |
| 30 | " | $-CH_2-CH_2Cl$ | $-CH_3$ | $-CH_3$ | O | |
| 31 | " | $-CH_2-CH=CH_2$ | $-CH_3$ | $-CH_3$ | O | Harz |
| 32 | " | Cyclohexyl | $-CH_3$ | $-CH_3$ | O | Harz |

| Bsp. Nr. | $R^1$ | $R^2$ | X | physikal. Konstante oder IR-Spektrum [$cm^{-1}$] |
|---|---|---|---|---|
| 33 | 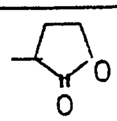 | $-CH_3$ | O | 3048,2950,1770,1735,1660, 1225,1160,1020,960,815,776 |
| 34 | " | $-C_2H_5$ | O | 3065,2985,1780,1743,1670, 1230,1172,1018,826,782 |
| 35 | " | $-C_3H_7-n$ | O | |
| 36 | " | $-C_3H_7-n$ | S | |
| 37 | " | $-C_3H_7-i$ | O | |
| 38 | " | $-C_4H_9-n$ | O | |
| 39 | " | $-C_4H_9-n$ | S | |
| 40 | " | $-C_4H_9-i$ | O | |
| 41 | " | $-C_4H_9-i$ | S | |
| 42 | " | $-C_4H_9$-sek. | O | |
| 43 | " | $-C_4H_9$-sek. | S | |
| 44 | " | $-C_4H_9$-tert. | O | |

| Bsp. Nr. | $R^1$ | $R^2$ | X | physikal. Konstante oder IR-Spektrum [$cm^{-1}$] |
|---|---|---|---|---|
| 45 | 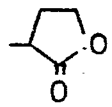 | $-CH_2-CH_2Cl$ | O | |
| 46 | " | $-CH_2-CH=CH_2$ | O | |
| 47 | " | $-CH_2CH=CHCH_3$ | O | |
| 48 | " | $-C_5H_{11}-n$ | O | |
| 49 | " | Cyclopentyl | O | |
| 50 | " | Cyclohexyl | O | |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podosphaera leucotricha an Äpfeln, Sphaerotheca fuliginea an Rosen, Erysiphe cichoracearum an Gurken. Die fungiziden Mittel enthalten 0,1 bis 95 % (Gew.%) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungen systemisch wirksam, so daß über die Wurzelbehandlung auch ein Schutz oberirdischer Pflanzenteile möglich ist.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangen-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin

3-(3,5-Dichlorphenyl)-5-methyl-5-methoxymethyl-1,3-oxazolidin-2,4-dion.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat) und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester

4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4--dioxid
2,3-Dihydro-5-carboxanilido-6-methyl-1,4-oxathiin
2-(Furyl-(2))-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Iod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)--2-butanon
1-(4-Chlorphenoxy)-3,3-dimethyl-1-(1H-1,2,4-triazol-1-yl)--2-butanol
alpha-(2-Chlorphenyl)-alpha-(4-chlorphenyl)-5-pyrimidin--methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Gießen angewendet. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel

oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatommeenerde, Calcium- und Magnesiumsulfat, Magnesiumoxid, gemahlene Kunststoffe,

Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Für die folgenden Versuche wurden als bekannte Vergleichswirkstoffe die folgenden Verbindungen verwendet:

N-Trichlormethylthio-phthalimid (Verbindung A)
N-Trichlormethylthio-tetrahydrophthalimid (Verbindung B)
Zink-ethylen-1,2-bis-dithiocarbamat (Verbindung C)

Versuch 1

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruchs abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise die Verbindungen Nr. 1, 2 und 4 bei Anwendung als 0,025%ige

Spritzbrühe eine bessere fungizide Wirkung (beispielsweise 97 %) zeigen als der bekannte Wirkstoff A (beispielsweise 90 %).

Versuch 2

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß die Verbindung Nr. 2 bei Anwendung als 0,025 %ige Spritzbrühe eine bessere fungizide Wirkung (beispielsweise 97 %) zeigt als die bekannten Wirkstoffe B und C (beispielsweise 70 %).

Beispiele für Zubereitungen sind:

I. Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung 2 werden in einer Mischung gelöst, die aus 80 Gewichtsteilen Xylol, 10 Gewichtsteilen des Anlagerungsproduktes von 8

bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 1 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser er--hält man eine wäßrige Dispersion.

IV. 20 Gewichtsteile der Verbindung 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser, erhält man eine wäßrige Dispersion.

V. 80 Gewichtsteile der Verbindung 2 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel·gut vermischt und in einer Hammermühle vermahlen. Durch feines Verteilen der Mischung in Wasser erhält man eine Spritzbrühe.

VI. 3 Gewichtsteile der Verbindung 4 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 4 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Patentansprüche

1. Oxalamid der allgemeinen Formel I

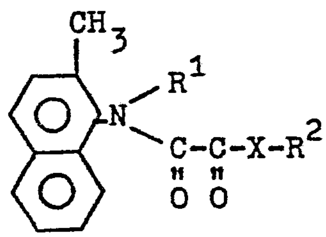

I,

worin

$R^1$ 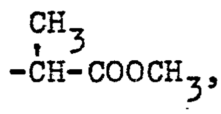, 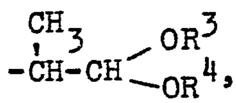, oder 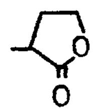 und

$R^2$ einen gegebenenfalls durch Methoxy, Ethoxy oder Halogen substituierten $C_1$-$C_6$-Alkyl-, $C_2$-$C_6$--Alkenyl- oder $C_5$-$C_6$-Cycloalkylrest bedeutet,

X für O oder S steht und

$R^3$ und $R^4$ Methyl oder Ethyl bedeuten oder $R^3$ und $R^4$ zusammen eine $C_2$-$C_3$-Alkylengruppe bedeuten, die zusammen mit dem Rest 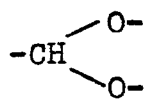 einen gegebenenfalls durch $C_1$-$C_3$-Alkyl substituierten Dioxolan- oder 1,3-Dioxanring bilden.

2. Oxalamid gemäß Anspruch 1, dadurch gekennzeichnet, daß $R^2$ Methyl, Ethyl, n-Propyl, Isopropyl, n-Butyl, Isobutyl, sek.-Butyl, tert.-Butyl, n-Pentyl, Isoamyl, 2-Methoxyethyl, 2-Ethoxyethyl, 3-Methoxypropyl, 2-Chlorethyl, 2-Bromethyl, 2,2,2-Trifluorethyl, Allyl, 2-Buten-1-yl, Cyclopentyl oder Cyclohexyl bedeutet.

3. Fungizid, enthaltend eine Verbindung gemäß Anspruch 1.

4. Fungizid, enthaltend ein Oxalamid gemäß Anspruch 1, dadurch gekennzeichnet, daß $R^2$ Methyl, Ethyl, n-Propyl, Isopropyl, n-Butyl, Isobutyl, sek.-Butyl, tert.-Butyl, n-Pentyl, Isoamyl, 2-Methoxyethyl, 2-Ethoxyethyl, 3-Methoxypropyl, 2-Chlorethyl, 2-Bromethyl, 2,2,2-Trifluorethyl, Ally, 2-Buten-1-yl, Cyclopentyl oder Cyclohexyl bedeutet.

5. Fungizide Mittel, enthaltend ein Oxalamid gemäß Anspruch 1 und einen inerten Trägerstoff.

6. Verfahren zur Bekämpfung von Pilzen, dadurch gekennzeichnet, daß man ein Oxalamid gemäß Anspruch 1 auf die Pilze oder auf durch Pilzbefall bedrohte Flächen, Pflanzen oder Saatgüter einwirken läßt.

7. Verfahren zur Herstellung von Oxalamiden gemäß Anspruch 1, dadurch gekennzeichnet, daß man ein 2-Methylnaphthylamin der Formel II

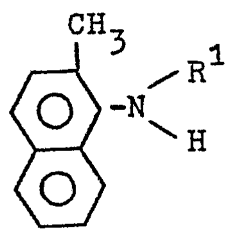

II,

worin

$R^1$ die in Anspruch 1 angegebene Bedeutung hat, mit einem Oxalhalbesterchlorid der Formel III

$$R^2 - X - \underset{\substack{\| \\ O}}{C} - \underset{\substack{\| \\ O}}{C} - Cl \qquad III,$$

worin
$R^2$ und X die in Anspruch 1 angegebene Bedeutung haben,

gegebenenfalls in Gegenwart eines Lösungsmittels, gegebenenfalls unter Zusatz einer organischen oder anorganischen Base und gegebenenfalls unter Zusatz eines Reaktionsbeschleunigers bei Temperaturen zwischen -10 und 100°C umsetzt.

## Patentansprüche (für Österreich)

1. Fungizid, enthaltend ein Oxalamid der allgemeinen Formel I

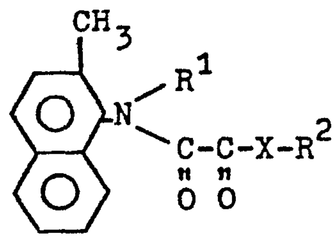 I,

worin

$R^1$ 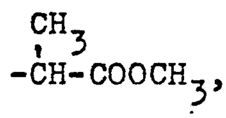, 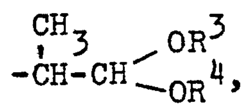, oder 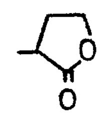 und

$R^2$ einen gegebenenfalls durch Methoxy, Ethoxy oder Halogen substituierten $C_1$-$C_6$-Alkyl-, $C_2$-$C_6$-Alkenyl- oder $C_5$-$C_6$-Cycloalkylrest bedeutet,

X für O oder S steht und

$R^3$ und $R^4$ Methyl oder Ethyl bedeuten oder $R^3$ und $R^4$ zusammen eine $C_2$-$C_3$-Alkylengruppe bedeuten, die zusammen mit dem Rest 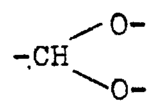 einen gegebenenfalls durch $C_1$-$C_3$-Alkyl substituierten Dioxolan- oder 1,3-Dioxanring bilden.

2. Fungizid, enthaltend ein Oxalamid gemäß Anspruch 1, <u>dadurch gekennzeichnet</u>, daß $R^2$ Methyl, Ethyl, n-Propyl, Isopropyl, n-Butyl, Isobutyl, sek.-Butyl, tert.-Butyl, n-Pentyl, Isoamyl, 2-Methoxyethyl, 2-Ethoxyethyl, 3-Methoxypropyl, 2-Chlorethyl, 2-Bromethyl, 2,2,2-Trifluorethyl, Ally, 2-Buten-1-yl, Cyclopentyl oder Cyclohexyl bedeutet.

3. Fungizide Mittel, enthaltend ein Oxalamid gemäß Anspruch 1 und einen inerten Trägerstoff.

4. Verfahren zur Bekämpfung von Pilzen, <u>dadurch gekennzeichnet</u>, daß man ein Oxalamid gemäß Anspruch 1 auf die Pilze oder auf durch Pilzbefall bedrohte Flächen, Pflanzen oder Saatgüter einwirken läßt.

5. Verfahren zur Herstellung von Oxalamiden gemäß Anspruch 1, <u>dadurch gekennzeichnet</u>, daß man ein 2-Methylnaphthylamin der Formel II

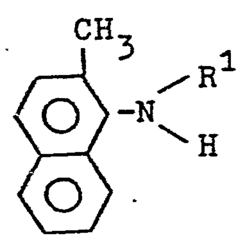 II,

worin

$R^1$ die in Anspruch 1 angegebene Bedeutung hat, mit einem Oxalhalbesterchlorid der Formel III

$$R^2 - X - \underset{\overset{\|}{O}}{C} - \underset{\overset{\|}{O}}{C} - Cl \qquad III,$$

worin
$R^2$ und X die in Anspruch 1 angegebene Bedeutung haben,

gegebenenfalls in Gegenwart eines Lösungsmittels, gegebenenfalls unter Zusatz einer organischen oder anorganischen Base und gegebenenfalls unter Zusatz eines Reaktionsbeschleunigers bei Temperaturen zwischen -10 und 100°C umsetzt.

Europäisches Patentamt

**0074071**
Nummer der Anmeldung

# EUROPÄISCHER RECHERCHENBERICHT

EP 82 10 8021

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | CH-A- 617 832 (CIBA-GEIGY) *Patentansprüche* | 1,3-6 | C 07 C 103/38<br>C 07 C 103/48<br>C 07 C 153/09<br>C 07 D 307/32<br>C 07 D 317/28<br>A 01 N 37/30<br>A 01 N 43/08 |
| Y | EP-A-0 009 569 (BAYER) *Patentansprüche* | 1,3-7 | |
| Y | EP-A-0 018 510 (CIBA-GEIGY) *Patentansprüche* | 1,3-6 | |
| Y | EP-A-0 029 996 (BASF) *Patentansprüche* | 1,3-6 | |

RECHERCHIERTE SACHGEBIETE (Int. Cl. 3)

C 07 C 103/00
C 07 C 153/00
C 07 D 307/00
A 01 N 37/00
A 01 N 43/00

Der vorliegende Recherchenbericht wurde für alle Patentansprüche erstellt.

| Recherchenort | Abschlußdatum der Recherche | Prüfer |
|---|---|---|
| DEN HAAG | 09-12-1982 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument